# EUROPEAN PATENT APPLICATION

(11) **EP 4 484 442 A1**
(43) Date of publication of application: **01.01.2025**
(21) Application number: 23182547.2
(22) Date of filing: 29.06.2023
(51) Int. Cl.: C07K 14/47, A61K 38/00, A61K 39/00, C12Q 1/6886, G01N 33/574

(54) **TISSUE EXTRACELLULAR PEPTIDE PATTERNS DEDICATED FOR GENERATION OF ANTI-CANCER VACCINES AGAINST SALIVARY GLAND TUMOR, IDENTIFICATION AND ISOLATION OF SALIVARY GLAND TUMOR SPECIFIC T CELLS IN HUMANS**

(71) Applicant: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Pirog, Artur, 80-312 Gdansk (PL); Faktor, Jakub, 80-170 Gdansk (PL); Czaplewska, Pauina, 80-461 Gdansk (PL)
(74) Representative: Czarnik, Maciej

(57) **Abstract**

The invention relates to 3 tissue extracellular peptide patterns dedicated for generation of anti-cancer vaccines against salivary gland tumor, identification and isolation of salivary gland tumor specific T cells in humans that are the list of the 52 quantitative peptides that in extracellular tumor tissue of patient with salivary gland tumor are increased 4 folds or more as compared with healthy tissue from the same patient. Therefore, this invention has significant implications for understanding fundamental and applied biological questions and generation of anti-cancer cellular therapies and peptide vaccines.

SEQUENCE LISTING

<110> Uniwersytet Gdanski

<120> Tissue extracellular peptide patterns dedicated for generation of anti-cancer vaccines against salivary gland tumor, identification and isolation of salivary gland tumor specific T cells in humans

<130>

<140>

<141>

<160> 52

<211> 14

<212> PRT

<213> Homo sapiens

<210> 1

<400> 1

Ser Thr Pro Leu Gly Gln Gln Gln Pro Ala Pro Arg Thr Arg

                5                   10

<211> 11

<212> PRT

<213> Homo sapiens

<210> 2

<400> 2

Ala Leu Arg Pro Ser Thr Ser Arg Ser Leu Tyr

                5                   10

<211> 13

<212> PRT

<213> Homo sapiens

<210> 3

<400> 3

Glu Thr Arg Asp Gly Gln Val Ile Asn Glu Thr Ser Gln

                5                   10

<211> 11

<212> PRT

<213> Homo sapiens

<210> 4

<400> 4

Ser Ser Leu Asn Leu Arg Glu Thr Asn Leu Asp

                5                   10

<211> 11

<212> PRT

<213> Homo sapiens

<210> 5

<400> 5

Thr Pro Leu Gly Gln Gln Gln Pro Ala Pro Arg
                5                   10

<211> 7
<212> PRT
<213> Homo sapiens
<210> 6
<400> 6

Ala Arg Met Leu Ala Gln Glu
                5

<211> 7
<212> PRT
<213> Homo sapiens
<210> 7
<400> 7

Ser Thr Ser Arg Ser Leu Tyr
                5

<211> 10
<212> PRT
<213> Homo sapiens

<210> 8

<400> 8

Leu Val Asp Thr His Ser Lys Arg Thr Leu

                5                   10

<211> 19

<212> PRT

<213> Homo sapiens

<210> 9

<400> 9

Phe Gly Gly Pro Gly Thr Ala Ser Arg Pro Ser Ser Ser Arg Ser Tyr

                5                   10                  15

Val Thr Thr

<211> 10

<212> PRT

<213> Homo sapiens

<210> 10

<400> 10

Thr Arg Asp Gly Gln Val Ile Asn Glu Thr

                5                   10

<211> 18

<212> PRT

<213> Homo sapiens

<210> 11

<400> 11

Gly Gly Pro Gly Thr Ala Ser Arg Pro Ser Ser Ser Arg Ser Tyr Val

                5                   10                  15

Thr Thr

<211> 22

<212> PRT

<213> Homo sapiens

<210> 12

<400> 12

Leu Ile Lys Thr Val Glu Thr Arg Asp Gly Gln Val Ile Asn Glu Thr

                5                   10                  15

Ser Gln His His Asp Asp

            20

<211> 14

<212> PRT

<213> Homo sapiens

<210> 13

<400> 13

Asp Ser Leu Pro Leu Val Asp Thr His Ser Lys Arg Thr Leu

                5                   10

<211> 9

<212> PRT

<213> Homo sapiens

<210> 14

<400> 14

Tyr Glu Leu Asn Pro Phe Ile Asn Arg

                5

<211> 16

<212> PRT

<213> Homo sapiens

<210> 15

<400> 15

Ser Pro Gly Gly Val Tyr Ala Thr Arg Ser Ser Ala Val Arg Leu Arg

                5                   10                  15

<211> 10

<212> PRT

<213> Homo sapiens

<210> 16

<400> 16

Asp Val Arg Gln Gln Tyr Glu Ser Val Ala

                5                   10

<211> 12

<212> PRT

<213> Homo sapiens

<210> 17

<400> 17

Ser Pro Gly Gly Val Tyr Ala Thr Arg Ser Ser Ala

                5                   10

<211> 12

<212> PRT

<213> Homo sapiens

<210> 18

<400> 18

Ser Thr Pro Leu Gly Gln Gln Gln Pro Ala Pro Arg
                5                   10
<211> 12
<212> PRT
<213> Homo sapiens
<210> 19
<400> 19
Val Glu Thr Arg Asp Gly Gln Val Ile Asn Glu Thr
                5                   10
<211> 8
<212> PRT
<213> Homo sapiens
<210> 20
<400> 20
Pro Gly Val Arg Leu Leu Gln Asp
                5
<211> 7
<212> PRT

<213> Homo sapiens

<210> 21

<400> 21

Asp Phe Ser Leu Ala Asp Ala

                5

<211> 10

<212> PRT

<213> Homo sapiens

<210> 22

<400> 22

Ser Ser Val Pro Gly Val Arg Leu Leu Gln

                5                   10

<211> 9

<212> PRT

<213> Homo sapiens

<210> 23

<400> 23

Thr Arg Asp Gly Gln Val Ile Asn Glu

                5

<211> 15

<212> PRT

<213> Homo sapiens

<210> 24

<400> 24

Gly Gly Pro Gly Thr Ala Ser Arg Pro Ser Ser Ser Arg Ser Tyr

                5                   10                  15

<211> 9

<212> PRT

<213> Homo sapiens

<210> 25

<400> 25

Ser Ser Leu Asn Leu Arg Glu Thr Asn

                5

<211> 12

<212> PRT

<213> Homo sapiens

<210> 26

<400> 26

Met His Lys Glu Glu His Glu Val Ala Val Leu Gly
                5                   10
<211> 20
<212> PRT
<213> Homo sapiens
<210> 27
<400> 27
Val Glu Thr Arg Asp Gly Gln Val Ile Asn Glu Thr Ser Gln His His
                5                   10                  15
Asp Asp Leu Glu
<211> 18
<212> PRT
<213> Homo sapiens
<210> 28
<400> 28
Gly Lys Val Gly Ala His Ala Gly Glu Tyr Gly Ala Glu Ala Leu Glu
                5                   10                  15
Arg Met

<211> 12

<212> PRT

<213> Homo sapiens

<210> 29

<400> 29

Ser Thr Arg Ser Val Ser Ser Ser Ser Tyr Arg Arg

                5                   10

<211> 11

<212> PRT

<213> Homo sapiens

<210> 30

<400> 30

Ser Ala Leu Arg Pro Ser Thr Ser Arg Ser Leu

                5                   10

<211> 8

<212> PRT

<213> Homo sapiens

<210> 31

<400> 31

Ser Leu Asn Leu Arg Glu Thr Asn
                5
<211> 8
<212> PRT
<213> Homo sapiens
<210> 32
<400> 32
Phe Thr Arg Glu Pro Gln Asp Thr
                5
<211> 7
<212> PRT
<213> Homo sapiens
<210> 33
<400> 33
Val Ser Lys Pro Asp Leu Thr
                5
<211> 12
<212> PRT
<213> Homo sapiens

<210> 34

<400> 34

Arg Gly Pro Glu Asp Glu Ile Gly Asp Pro Leu Ala

                5                   10

<211> 12

<212> PRT

<213> Homo sapiens

<210> 35

<400> 35

Ser Ser Val Pro Gly Val Arg Leu Leu Gln Asp Ser

                5                   10

<211> 12

<212> PRT

<213> Homo sapiens

<210> 36

<400> 36

Val Val Tyr Pro Trp Thr Gln Arg Phe Phe Glu Ser

                5                   10

<211> 11

<212> PRT

<213> Homo sapiens

<210> 37

<400> 37

Phe Leu Ser Phe Pro Thr Thr Lys Thr Tyr Phe

                5                   10

<211> 13

<212> PRT

<213> Homo sapiens

<210> 38

<400> 38

Asp Leu Glu Pro Thr Val Ile Asp Glu Val Arg Thr Gly

                5                   10

<211> 16

<212> PRT

<213> Homo sapiens

<210> 39

<400> 39

Tyr Gln Lys Val Val Ala Gly Val Ala Asn Ala Leu Ala His Lys Tyr

                5                   10                  15

<211> 11

<212> PRT

<213> Homo sapiens

<210> 40

<400> 40

Ser Ser Val Pro Gly Val Arg Leu Leu Gln Asp

                5                   10

<211> 12

<212> PRT

<213> Homo sapiens

<210> 41

<400> 41

Asp Asp Val Val Gly Glu Thr Val Gly Lys Thr Asp

                5                   10

<211> 12

<212> PRT

<213> Homo sapiens

<210> 42

<400> 42

Asp Ser Leu Pro Leu Val Asp Thr His Ser Lys Arg
                5                   10

<211> 16
<212> PRT
<213> Homo sapiens
<210> 43
<400> 43

Ser Pro Ala Ala Lys Pro Gly Ser Thr Pro Ser Arg Pro Ser Ser Ala
                5                   10                  15

<211> 7
<212> PRT
<213> Homo sapiens
<210> 44
<400> 44

Glu Tyr Val Gln Thr Val Lys
                5

<211> 14

<212> PRT

<213> Homo sapiens

<210> 45

<400> 45

Gln Val Ile Asn Glu Thr Ser Gln His His Asp Asp Leu Glu

                5                   10

<211> 8

<212> PRT

<213> Homo sapiens

<210> 46

<400> 46

Phe Ile Glu Asn Glu Glu Gln Glu

                5

<211> 7

<212> PRT

<213> Homo sapiens

<210> 47

<400> 47

Ala Ile Gln Phe Gly Asn Tyr

                5

<211> 10

<212> PRT

<213> Homo sapiens

<210> 48

<400> 48

Thr His Phe Asn Lys Gly Pro Ser Tyr Gly

                5                   10

<211> 10

<212> PRT

<213> Homo sapiens

<210> 49

<400> 49

Ser His Ser Leu Thr Thr Asn Ile Met Glu

                5                   10

<211> 9

<212> PRT

<213> Homo sapiens

<210> 50

<400> 50

Val Pro Val Glu Ala Val Thr Ser Lys
                5
<211> 20
<212> PRT
<213> Homo sapiens
<210> 51
<400> 51
Ala Ala Asp Pro Pro Ala Glu Asn Ser Ser Ala Pro Glu Ala Glu Gln
                5                   10                  15
Gly Gly Ala Glu
            20
<211> 26
<212> PRT
<213> Homo sapiens
<210> 52
<400> 52
Ser Glu Ser Gly Ser Phe Arg Pro Asp Ser Pro Gly Ser Gly Asn Ala
                5                   10                  15
Arg Pro Asn Asn Pro Asp Trp Gly Thr Phe

20                    25

## Description

The invention refers to tissue extracellular peptide peptides grouped into patterns dedicated for generation of anti-cancer vaccines against salivary gland tumor, identification and isolation of salivary gland tumor specific T cells in humans.

The causes of salivary gland tumors are often unknown, and a proper evaluation by medical professionals is necessary to diagnose them. This includes physical examination and imaging tests like ultrasound, computerized tomography (CT) scan, or magnetic resonance imaging (MRI). Early screening and diagnosis using extracellular tissue peptidomics is crucial in developing effective therapy not just for salivary gland tumors but also for a wide range of disease models.

The MS-based discovery proteomics has reported several protein markers [1] The proteins ANXA1(Annexin A1), ANXA5 (Annexin A5), CRYAB (α-Crystallin), FGB (Fibrinogen), PPIA (Peptidyl-prolyl cis/trans isomerases) were proposed as the most common potential diagnostic markers of salivary gland tumors. Moreover, the imaging studies and immunohistochemistry combined with hematoxylin and eosin staining procedures remain the gold standard of salivary gland pathology. Many studies demonstrate developments in histochemical diagnostics and translocations for differentiating [2] salivary gland tumors. They also suggested that, in time, several novel molecular alterations are expected to be discovered, which might potentially increase the importance of markers. For the first time, the inventors have proposed peptide pattern markers are the source of tissue extracellular peptides and not the protein marker. The invention is simple, cost-effective, fast, and comprehensive for solid tumor peptidomics profiling. Moreover, the methods enable the discovery of complex extracellular peptidomics that can be used for generation of anti-cancer vaccines against salivary gland tumor, identification and isolation of salivary gland tumor specific T cells in humans.

Several protein markers have been identified through MS-based discovery proteomics. The proteins ANXA1(Annexin A1), ANXA5 (Annexin A5), CRYAB (α-Crystallin), FGB (Fibrinogen), PPIA (Peptidyl-prolyl cis/trans isomerases) [1] have been suggested as the most common potential diagnostic markers for salivary gland tumors. Imaging studies and immunohistochemistry, combined with hematoxylin and eosin staining procedures, are still considered the gold standard for salivary gland pathology. Many studies have demonstrated advancements in histochemical diagnostics and translocations [2] to differentiate salivary gland tumors. It has been suggested that novel molecular alterations may potentially increase the importance of markers in the future. The inventors have proposed that peptide pattern dedicated for generation of anti-cancer vaccines against salivary gland tumor, identification and isolation of salivary gland tumor specific T cells in humans originate from tissue extracellular peptides rather than protein markers. This invention is simple, cost-effective, fast, and comprehensive in solid tumor peptidomics profiling. Additionally, This invention method enable the discovery of complex extracellular peptidomics that can be used for biomarker discovery, designing novel immunotherapy approaches. The concept of peptidomics has been known for at least 20-30 years. It was first applied to specific classes of bioactive peptides, such as neuropeptides. Later, with the development of analytical technology, peptides were analyzed in body fluids and various secretions. The peptidome [3] is considered as low molecular weight peptides/proteins (less than 15 kilodaltons (kDa). The tissue extracellular peptidome has a diverse range of peptides and their potential roles in various physiological and pathological processes (cell-cell communication, signaling). Tissue extracellular peptidome analysis primarily focuses on analyzing peptides present in the extracellular space. Hence these peptides indicate various biological processes, such as cell communication, disease processes, and reflect the health or disease state of the person. In addition, it provides insight into biomarker and therapeutic target discovery and drug developments, allowing the study of disease microenvironments.

When someone has an abnormal salivary gland, it may be due to a salivary gland tumor. To diagnose this condition, several steps are typically involved, including medical history and physical examination, imaging tests, biopsy, pathology examination, and more.

Unfortunately, all of these diagnostic approaches are time-consuming and expensive. However, a new method called tissue extra peptidomics has been developed, which can reveal 52 peptides dedicated for generation of anti-cancer vaccines against salivary gland tumor, identification and isolation of salivary gland tumor specific T cells in humans in a quantifiable way. There are several methods available to accurately diagnose salivary gland tumors, including physical examinations and imaging techniques like ultrasound and Magnetic Resonance Imaging (MRI). These methods can identify masses, cysts, and the size and location of the tumor. Additionally, Positron Emission Tomography (PET) or Computed Tomography (CT) scans can help evaluate the spread of cancer. However, these methods can be expensive and require appointments with specialists. Medical specialists recommend combining multiple diagnostic methods for a comprehensive evaluation, which often leads to accurate diagnosis and appropriate management of salivary gland disorders. Nonetheless, accurate diagnosis for salivary gland tumors is lacking. Our approach involves identifying multi-biomarkers of naturally occurring tissue extracellular peptides from salivary gland tumor tissue samples. Data from previous literature [1] showed that the protein biomarker approach had been used for the diagnosis of various ranges of diseases. However, the ultimate success of these previously published methods is severely limited due to the low number of identified protein markers.

This invention introduces a unique method for preparing tissue extracellular peptidomics for qualitative and quantitative analysis. The use of tissue extracellular peptides for early screening and diagnosis is crucial in developing effective therapies for various diseases. While protein biomarkers are widely used to diagnose tumors, they have some limitations, such as lack of specificity, many protein biomarkers are not specific to a single disease or condition and can be present in multiple diseases or healthy individuals, resulting in false positives or false negatives. Additionally, protein biomarker levels can vary significantly among individuals, even within the same disease category, due to inter-individual variability. Protein biomarkers may have limited sensitivity and dynamic ranges, making them undetectable at early stages of a disease or in low concentrations. Additionally, pre-analytical variables such as sample collection, handling, and storage can affect the measurement of protein biomarkers. The cost and time required for developing and validating protein biomarker assays can limit their widespread availability and affordability, particularly in areas with limited access to advanced laboratory facilities. However, the invention of tissue extracellular peptidomics, which sequences natural tissue extracellular peptides without modifying them during sample preparation, offers a novel approach with technological advancements to address these limitations. This aims to improve the accuracy, sensitivity, and specificity of protein biomarker-based diagnostics and therapies.

Tissue peptidomics is still a developing field, the nature and complexity of tissue extracellular peptidome are currently not well defined. In the past, there is no consensus on the expected yield of tissue extracellular peptide isolation. This suggests that considerable sample losses are affecting reported methods. In the inventors opinion, minimum steps of sample preparation allow to comprehensive yield and biomarker discovery from tissue extracellular peptidome. Moreover the cell lysis, while using only one type of denaturing agent [4] cannot provide optimal detection of biomarker peptides. One major difference is that our focus is on the tissue extracellular peptidome, rather than just proteins. Our peptide patterns are carefully selected through both qualitative and quantitative analysis, with only quantitative markers being considered. In contrast, other methods, such as [5], [6], [7], primarily focus on identifying proteins and peptides. The tissue extracellular markers inventors listed for salivary gland peptide patterns dedicated for generation of anti-cancer vaccines against salivary gland tumor, identification and isolation of salivary gland tumor specific T cells in humans are particularly unique because they require no additional manipulation steps, such as reduction, alkylation, or enzymatic (tryptic) digestion during the sample preparation. This allows us to identify natural tissue extracellular peptides without any modifications.

The invention refers to tissue extracellular peptide patterns dedicated for generation of anti-cancer vaccines against salivary gland tumor, identification and isolation of salivary gland tumor specific T cells in humans. The invention is simple, cost-effective, fast, and comprehensive for solid tumor peptidomics profiling. Moreover, the methods enable the discovery of complex extracellular peptidomics that can be used for patterns dedicated for generation of anti-cancer vaccines against salivary gland tumor, identification and isolation of salivary gland tumor specific T cells in humans. Hence inventions provide valuable insights for understating the basic and applied biological questions and designing novel immunotherapy approaches.

The invention is related to the tissue extracellular peptide patterns dedicated for generation of anti-cancer vaccines against salivary gland tumor, identification and isolation of salivary gland tumor specific T cells in humans. The invention is based on the identification (qualitative analysis) and relative quantification (quantitative analysis) of a unique for salivary gland tumor multi-biomarker panel of tissue extracellular peptides (identified based on amino acid sequences). 52 of peptides patterns dedicated for generation of anti-cancer vaccines against salivary gland tumor, identification and isolation of salivary gland tumor specific T cells in humans are increased 4 folds or more in the extracellular tissue of the patient with salivary gland tumor as compared with the healthy tissue of the same patient (quantitative analysis), As grade of the fold increase of the quantitative peptides correlates positively with their predictive significance for salivary gland tumor diagnostics, inventors also define a tissue extracellular peptide patterns 1, 2, and 3. The extracellular tissue tumor peptide pattern 1 is the list of 2 extracellular tissue peptides that are equal to or higher than 100 folds in tissue salivary gland tumor patients compared to healthy tissue from the same patient. The extracellular tissue tumor peptide pattern 2 is the list of 14 extracellular tissue peptides that are equal to or higher than 20 folds in tissue salivary gland tumor patients compared to healthy tissue from the same patient. The extracellular tissue tumor peptide pattern 3 is the list of 36 extracellular tissue peptides that are equal to or higher than 4 folds in tissue salivary gland tumor patients compared to healthy tissue from the same patient. Each of three tumor tissue extracellular peptide patterns can be used as a separate independent panel of markers patterns dedicated for generation of anti-cancer vaccines against salivary gland tumor, identification and isolation of salivary gland tumor specific T cells in humans and differentiation from healthy tissues and monitoring salivary gland tumor and patient response to the applied treatment.

Tissue invention is a group of 52 extracellular peptides in total divided on three patterns according to fold changes as follow
pattern 1: 2 tissue extracellular peptides with 100 folds,
pattern 2: 14 tissue extracellular peptides with 20 folds,
pattern 3: 36 tissue extracellular peptides with 4 fold so 2 tissue extracellular peptides with 100 folds, 14 tissue extracellular peptides with 20 folds, 36 tissue extracellular peptides with 4 folds. The peptides patterns dedicated for generation of anti-cancer vaccines against salivary gland tumor, identification and isolation of salivary gland tumor specific T cells in humans and differential diagnosis of salivary gland tumor from healthy tissues

Invention has comprehensive both qualitative and quantitative tissue extracellular peptidomics analysis, Moreover, the quantitative analysis of at least 9329 of the extracellular tissue peptidome is quantified and sequenced with a tandem mass spectrometry can be performed when the signal intensity of the studied peptides from healthy tissues and salivary gland tumor tissues are at least 4 fold higher (≥4) than the signal intensity of the matched control tissue samples. Salivary gland tumor extracellular tissue peptidomics pattern for patterns dedicated for generation of anti-cancer vaccines against salivary gland tumor, identification and isolation of salivary gland tumor specific T cells in humans that is a list of the following at least 52 quantitative peptides divided into patterns:

Tissue extracellular peptide patterns which can be targets for anti-cancer antigen specific T cells and also serve for diagnostics of salivary gland extracellular tumor in humans and differential diagnosis from healthy tissues that is a list of the following 52 quantitative peptides Ser-Thr-Pro-Leu-Gly-Gln-Gln-Gln-Pro-Ala-Pro-Arg-Thr-Arg, Ala-Leu-Arg-Pro-Ser-Thr-Ser-Arg-Ser-Leu-Tyr, Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala, Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of mentioned 52 peptides in extracellular tumor tissue of patient with salivary gland tumor are increased 4 folds or more as compared with healthy tissue from the same patient.

The tissue extracellular tumor peptide pattern, wherein tissue extracellular tumor peptide levels of the following 2 quantitative peptides Ser-Thr-Pro-Leu-Gly-Gln-Gln-Gln-Pro-Ala-Pro-Arg-Thr-Arg, and Ala-Leu-Arg-Pro-Ser-Thr-Ser-Arg-Ser-Leu-Tyr wherein the tissue extracellular tumor peptide level of mentioned 2 peptides in extracellular tumor tissue of patient with salivary gland tumor are increased 100 folds or more as compared with healthy tissue from the same patient.

The tissue extracellular tumor peptide pattern, wherein tissue extracellular tumor peptide levels of the following 14 quantitative peptides Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala wherein the tissue extracellular tumor peptide level of mentioned 14 peptides in extracellular tumor tissue of patient with salivary gland tumor are increased 20 folds or more as compared with healthy tissue from the same patient.

The tissue extracellular tumor peptide pattern, wherein tissue extracellular tumor peptide levels of the following 36 quantitative peptides Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of mentioned 36 peptides in extracellular tumor tissue of patient with salivary gland tumor are increased 4 folds or more as compared with healthy tissue from the same patient. As grade of the fold increase of the quantitative peptides correlates positively with their predictive significance for salivary gland tumor for anti-cancer antigen specific T cells and diagnostics, inventors also define a tissue extracellular peptide patterns 1, 2, and 3. Each of three tumor tissue extracellular peptide patterns can be used as a separate independent panel of markers for anti-cancer antigen specific T cells and diagnostics of salivary gland tumor and differentiation from healthy tissues and monitoring salivary gland tumor and patient response to the applied treatment. Thus these 52 biomarker peptides play important roles in the accurate diagnosis and appropriate management of salivary gland tumor. It will revolutionize the for anti-cancer antigen specific T cells and early diagnostic field and show a roadmap to the Point of Care Diagnostic (POCD) developments. Ultimately, it will improve the quality of life and be applied to the globe. Therefore, the invention will bring breakthroughs in Point of Care Diagnostic (POCD) platforms and revolutionize the medical field. It is ready to be employed for all kinds (human/non-human) of disease tissue samples in the future.

### Example 1

The invention method is novel, simple, and it has only two steps sample preparation s that provides the screening and quantification of tissue extracellular peptides. Prior to starting any sample preparation steps of the tissue extracellular peptidomics method, it is strongly recommended to prepare all the solutions using LC-MS (Liquid Chromatography with mass spectrometry) grade water, solvents, reagents, and ultraclean glass and plasticware use during methods. Moreover, all solutions and buffers should be prepared immediately before use and disposed of after one week of storage. The first step is to prepare all the necessary buffers on the same day of tissue peptidomics samples preparation.

### Buffer preparations

1^{st} buffer: citrate-phosphate buffer at pH 3.1 to 3.6 - (0.131 M citric acid/0.066 M Na2HPO4, NaCl 150 mM) and adjust the pH 3.1 to 3.6 with 1M NaOH prepared in LC-MS (Liquid Chromatography with mass spectrometry) grade water.

2^{nd} solution, 0.2% formic acid/methanol volume per volume (v/v), was prepared (the final proportion of formic acid in methanol was 0.2%).

3^{rd} solution, 0.2% formic acid/water volume per volume (v/v), was prepared (the final proportion of formic acid in water was 0.2%).

4^{th} solution wash buffer, water/5% methanol/0.2% formic acid volume per volume (v/v), was prepared (the final proportion of methanol and formic acid in water was 5% and 0.2%, respectively).

5^{th} solution elution buffer, water/80% methanol/0.2% formic acid volume per volume (v/v), was prepared (the final proportion of methanol and formic acid in water was 80% and 0.2%, respectively).

Efforts were made to develop a repeatable and effective method to obtain a sufficient amount of sample from the smallest possible amount of tissue material.

Workflow has two steps sample preparation, a) extraction b) purification of tissue peptides.
a. extraction of extracellular tissue peptidome

Prior the extraction, the tissue samples are subjected to 2-3 washes with phosphate-buffered saline (PBS) without additives (no calcium/magnesium/phenol red) pH 7.2-7.5 and centrifuged at 500 ×g for 5 minutes at 4 °C. Then test tissue samples are incubated with an extraction buffer (citrate-phosphate buffer) with pH 3.1-3.6 prepared in liquid chromatography-mass spectrometry (LC-MS) grade ultrapure water, purity ≥ 99.9%, wherein the buffer is at a volume of 7-8 ml to 0.05-1 gram of tissue sample. The incubation step takes 2-3 minutes with mixing at a temperature of 4 to 10 °C to dissociate peptides from the highly concentrated tissue membrane surface and to collect the extracellular tissue peptidome by centrifuging at 500 ×g for 5 minutes at 4 °C. Therefore, it is characterized by the unexpected use of low pH buffer for the enrichment of tissue extracellular peptides and release from complexes from tissue surface;
b. purification of extracellular tissue peptidome

Collected supernatants for both samples set were subjected to the Oasis cartridges (30 mg; Waters, hydrophilic-lipophilic balanced columns) to perform reverse-phase purification of polypeptides. All steps were performed at room temperature using only gravity flow, and all the detailed steps are as follows.
i) Cartridge's condition: cartridge was conditioned with 0.2% formic acid/methanol volume per volume (v/v) (in this solution final proportion of formic acid in methanol was 0.2%) twice, volume for each wash was 1ml/cartridge.
ii) Equilibration of cartridge: the cartridge was equilibrated with 0.2% formic acid/water volume per volume (v/v) (in this solution final proportion of formic acid in water was 0.2%), volume for each wash was 1ml/cartridge.
iii) Sample loading: 6-8 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) was eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively),

Extracellular tissue peptidome, all filtration unit conditioning and filtration steps were performed immediately after elution. According to the instructions, to eliminate proteins equal or bigger than 15-30 kDa from peptide enriched and simultaneously elute the tissue extracellular peptides further separated with an ultrafiltration using selected molecular weight cutoff filter that has 3-30 kilo daltons (kDa), preferably 3 or 10 or 30, kDa for 120-135 minutes centrifuge at 4000 g, at 4 - 10 °C and removal of high- molecular weight proteins and other cellular compounds that have > 30 kDa; Lastly, the pipette eluted and diluted peptide solution (2ml) into the device, taking care not to touch the membrane with the pipette tip. Collect the filtrate (extracellular tissue peptidome), then evaporate it to dryness. All dried peptide samples were stored at -80 until the mass spectrometry analysis.

### Example 2

Tissue extracellular peptidomics, the next crucial steps in the invention are mass spectrometry based measurements such as DDA (data-dependent acquisition) and DIA - (data-independent acquisition) mode. Mass spectrometry combined with chromatographic separation is necessary to identify several thousand peptides. Due to the unspecific nature of tissue extracellular peptides, successful identification requires the order of magnitude higher data quality than a standard trypsin-based proteomic sample, especially in terms of mass accuracy. Only recently, technological advances in mass spectrometry enabled the collection of data of quality acceptable for such analyses. DIA - (data-independent acquisition) method enables to the collection of quantitative data for all peptides present in the sample while maintaining good resilience to matrix interferences.

### Tissue extracellular peptidomics LC-MS/MS (Liquid Chromatography with tandem mass spectrometry) analysis

LC conditions were identical for DDA and DIA experiments. Samples were separated on the Thermo Scientific RSLC3000nano LC system, coupled to Thermo Scientific Orbitrap Exploris 480 mass spectrometer. Chromatography was performed by concentrating peptides on 0.3x5mm C18 PepMap trap column (Thermo Scientific) at the flow of 5µl/min for 10min and further separation on 0.075mm × 250mm 2µm C18 PepMap RSLC column (Thermo Scientific). The loading buffer was identical to the sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 35% in 80 minutes, followed increase to 60% in 15 minutes, washing with 99% B, and re-equilibration to initial conditions.

DDA data were acquired using a full scan with 120k resolution, 350-1650 Th mass range, 300% AGC (automatic gain control) target, and automatic maximum injection time. Full scans were saved as profile spectra. For fragmentation minimum intensity of 3000 was selected and dynamic exclusion was set to 20s within the +/-10ppm range from fragmented precursor ant its isotopes. The charge states 1 to 6 were allowed for fragmentation. Ion isolation window was set to 1.6 Th, resolution to 60k and normalized HCD (higher energy collisional dissociation) fragmentation energy to 30%. AGC target was set to Standard and maximum ion injection time to Auto. The fragment mass range was set to 'Define first mass' with starting m/z of 110 Th. Spectra were saved in profile mode. DIA data were acquired using full scan with 60k resolution, 350-1450 Th mass range, 300% AGC target, and 100ms maximum injection time. DIA used 62 12 Th windows with 1 Th overlaps, covering a mass range from 350 to 1100 Th. The resolution was set o 30k, AGC to 1000%, and HCD fragmentation energy to 30%, using 3+ as the default ion charge state. Spectra were saved in profile mode.

### Tissue extracellular peptide identification

Data were analyzed mostly with the goal of quantitative characterization of intracellular peptidome. Raw data files (DDA and DIA runs) were converted to .mzml files using MSConvert with centroiding. Converted MS data were searched using MSFragger 3.4 embedded in Fragpipe suite against the complete Homo sapiens Uniprot database (SwissProt+Trembl, all isoforms) concatenated with indexed retention time (iRT) peptide sequences and decoy reverse sequences and contaminants. The search database was constructed in Fragpipe v. 15.0 suite. Additional DDA-like data was extracted from DIA (data-independent acquisition) files by the DIA-Umpire signal extraction engine integrated with Fragpipe. The following search settings were used for MSFragger: precursor mass tolerance was set to +/-8parts per million (ppm), and fragment mass tolerance was automatically optimized. Enzyme digestion was set to non-specific and peptide length was set from 7 to 45 amino acids. The peptide mass range was set from 500 to 5000 Dalton. Variable modifications were set to: methionine oxidation, protein N-term acetylation. Data were mass recalibrated, and automatic parameter optimization setting was used to tune fragment mass tolerance. Search result filtration and validation were done automatically by Philosopher. EasyPQP were used to create the spectral libraries for further use in DIA-based quantification.

### Tissue extracellular peptide quantification

DIA data processing were performed using DIA-NN v. 1.8 All .mzml files were converted to .dia files. MBR option was selected. Precursor FDR was set to 1 %. Robust LC (high accuracy) option was selected. MS2 and MS1 mass accuracies, and scan window were automatically adjusted for each run. Retention time (RT)-dependent cross-run normalization was applied to data. Other settings in the tab were left default. Peptide quantification data were prepared by the diann-r R library. Data were preprocessed in Python to average technical replicates. Statistical analysis was performed using Perseus v.1.6.15. Peptide quantification containing more than one missing value was removed. Regulated peptides were identified using Student's test p-value cutoff of 0.01, and log2 fold change cutoff of 2.

The next part of the invention of tissue extracellular peptidome focuses on the discovery of extracellular biomarker peptides for peptide patterns dedicated for generation of anti-cancer vaccines against salivary gland tumor, identification and isolation of salivary gland tumor specific T cells in humans and salivary gland tumor early diagnostics. However, prior to do biomarker discovery, the inventors performed quality control check experiments and make sure that there is no interference of major histocompatibility complex (MHC) class I peptidome with tissue extracellular peptidome as these both are present on the tissue/cell surface. The first quality control, the inventors check of tissue extracellular peptidome extracts, are significantly differs than MHC ligand peptides. Tissue extracellular peptides are subjected to NetMHCpan server where predicts the binding of peptides to any MHC molecule of known sequence using artificial neural networks (ANNs). These peptides are categorized as all peptides, binders and strong binders peptides to MHC supertype representative. The yield of tissue extracellular peptides differ from MHC class I peptide has been confirm by comparing the detected peptides and random/artificial peptides. Tissue peptidome extract does not contain a significant amount of MHC ligand peptides. In analyzed peptide samples, only 671 out of 9321 (7.2%) peptides show potential for MHC binding, which is even less than the result for the random peptide set (799 predicted binders (8.5%). Thus, NetMHCpan binding of peptides predictions revealed that tissue extracellular peptidome shows a less percentage of MHC binding than random/artificial peptide set predicted MHC binders. Also, length distribution presented do not show the typical of MHC class I peptide enrichment of 9-mer peptides only. This strongly suggests that detected peptides represent a broad population of tissue extracellular peptidome rather than a subpopulation of MHC class I peptide.

For further analysis, the invention of tissue extracellular peptidome considered only tissue extracellular peptides fully quantified in the salivary gland tumor tissue (n=4) and healthy tissue (n=4) samples. It has shown the identification with excellent reproducibility. Statistical analysis was performed using Perseus v.1.6.15. Peptide quantification containing more than one missing value was removed. Quantification data obtained by DIA-NN were post-processed in Perseus. Significantly regulated tissue extracellular peptides were selected by using Student's test p-value cutoff of 0.01, log2 fold change cutoff of 2 and permutation-based FDR (false discovery rate) correction at 1% FDR. Tissue extracellular peptide quantitation experiment provides a list of peptides that significantly stratify between compared groups where quantitative analysis of the extracellular tissue peptides sequenced with a tandem mass spectrometry can be performed when signal intensity of the studied peptide is at least 4 fold higher (≥4) than the signal intensity of the matched healthy tissue samples. The quantitative analysis of at least 9329 of the extracellular tissue peptidome is quantified and sequenced with a tandem mass spectrometry can be performed when the signal intensity of the studied peptides from healthy tissues (n=4) and salivary gland tumor tissues (n=4) are at least 4 fold higher (≥4) than the signal intensity of the matched healthy tissues samples. Salivary gland tumor extracellular tissue peptidomics pattern dedicated for generation of anti-cancer vaccines against salivary gland tumor, identification and isolation of salivary gland tumor specific T cells in humans and for diagnostics of salivary gland tumor that is a list of the following at least 52 quantitative peptides:
Tissue extracellular peptide patterns which can be targets for anti-cancer antigen specific T cells and also serve for diagnostics of salivary gland extracellular tumor in humans and differential diagnosis from healthy tissues that is a list of the following 52 quantitative peptides Ser-Thr-Pro-Leu-Gly-Gln-Gln-Gln-Pro-Ala-Pro-Arg-Thr-Arg, Ala-Leu-Arg-Pro-Ser-Thr-Ser-Arg-Ser-Leu-Tyr, Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala, Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of mentioned 52 peptides in extracellular tumor tissue of patient with salivary gland tumor are increased 4 folds or more as compared with healthy tissue from the same patient.

The tissue extracellular tumor peptide pattern, wherein tissue extracellular tumor peptide levels of the following 2 quantitative peptides Ser-Thr-Pro-Leu-Gly-Gln-Gln-Gln-Pro-Ala-Pro-Arg-Thr-Arg, and Ala-Leu-Arg-Pro-Ser-Thr-Ser-Arg-Ser-Leu-Tyr wherein the tissue extracellular tumor peptide level of mentioned 2 peptides in extracellular tumor tissue of patient with salivary gland tumor are increased 100 folds or more as compared with healthy tissue from the same patient.

The tissue extracellular tumor peptide pattern, wherein tissue extracellular tumor peptide levels of the following 14 quantitative peptides Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala wherein the tissue extracellular tumor peptide level of mentioned 14 peptides in extracellular tumor tissue of patient with salivary gland tumor are increased 20 folds or more as compared with healthy tissue from the same patient.

The tissue extracellular tumor peptide pattern, wherein tissue extracellular tumor peptide levels of the following 36 quantitative peptides Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of mentioned 36 peptides in extracellular tumor tissue of patient with salivary gland tumor are increased 4 folds or more as compared with healthy tissue from the same patient. As grade of the fold increase of the quantitative peptides correlates positively with their predictive significance for salivary gland tumor diagnostics, inventors also define a tissue extracellular peptide patterns 1, 2, and 3. Each of three tumor tissue extracellular peptide patterns can be used as a separate independent panel of markers peptide patterns dedicated for generation of anti-cancer vaccines against salivary gland tumor, identification and isolation of salivary gland tumor specific T cells in humans and for diagnostics of salivary gland tumor and differentiation from healthy tissues and monitoring salivary gland tumor and patient response to the applied treatment. Thus these 52 biomarker peptides play important roles in the accurate diagnosis and appropriate management of salivary gland tumor.

In the salivary gland tumor, extracellular peptidome higher fold change peptide sequences, and their source of the proteins identifier for each peptide is listed in Tables 1, 2, and 3, respectively. The fold change refers only to tumor extracellular quantitative peptides. It shows how many times signaling intensities in the salivary gland tumor samples were higher than in healthy tissue samples.

**Table 1. The extracellular tissue peptide sequences and their source of the proteins identifier for each peptide is listed. Two extracellular tissue peptides are equal to or higher than 100 folds (^{∗∗∗}) in tissue salivary gland tumor patients compared to healthy tissue from the same patient.**

| Serial number | Source protein identifier | Peptide sequence | Quantitative peptides Fold change*** ≥ 100 |
|---|---|---|---|
| 1 | Latent-transforming growth factor beta-binding protein 2 | Ser-Thr-Pro-Leu-Gly-Gln- Gln-Gln-Pro-Ala-Pro-Arg- Thr-Arg | |
| 2 | Vimentin | Ala-Leu-Arg-Pro-Ser-Thr-Ser- Arg-Ser-Leu-Tyr | |

**Table 2. The extracellular tissue peptide sequences and their source of the proteins identifier for each peptide is listed. Fourteen extracellular tissue peptides are equal to or higher than 20 folds (^{∗∗}) in tissue salivary gland tumor patients compared to healthy tissue from the same patient.**

| Serial number | Source protein identifier | Peptide sequence | Quantitative peptides Fold change** ≥ 20 |
|---|---|---|---|
| 3 | Vimentin | Glu-Thr-Arg-Asp-Gly-Gln-Val- Ile-Asn-Glu-Thr-Ser-Gln | |
| 4 | Vimentin | Ser-Ser-Leu-Asn-Leu-Arg-Glu- Thr-Asn-Leu-Asp | |
| 5 | Latent-transforming growth factor beta-binding protein 2 | Thr-Pro-Leu-Gly-Gln-Gln-Gln- Pro-Ala-Pro-Arg | |
| 6 | Calmodulin-like protein 5 | Ala-Arg-Met-Leu-Ala-Gln-Glu | |
| 7 | Vimentin | Ser-Thr-Ser-Arg-Ser-Leu-Tyr | |
| 8 | Vimentin | Leu-Val-Asp-Thr-His-Ser-Lys- Arg-Thr-Leu | |
| 9 | Vimentin | Phe-Gly-Gly-Pro-Gly-Thr-Ala- Ser-Arg-Pro-Ser-Ser-Ser-Arg-Ser- Tyr-Val-Thr-Thr | |
| 10 | Vimentin | Thr-Arg-Asp-Gly-Gln-Val-Ile- Asn-Glu-Thr | |
| 11 | Vimentin | Gly-Gly-Pro-Gly-Thr-Ala-Ser- Arg-Pro-Ser-Ser-Ser-Arg-Ser-Tyr- Val-Thr-Thr | |
| 12 | Vimentin | Leu-Ile-Lys-Thr-Val-Glu-Thr- Arg-Asp-Gly-Gln-Val-Ile-Asn- Glu-Thr-Ser-Gln-His-His-Asp- Asp | |
| 13 | Vimentin | Asp-Ser-Leu-Pro-Leu-Val-Asp- Thr-His-Ser-Lys-Arg-Thr-Leu | |
| 14 | Matrix Gla protein | Tyr-Glu-Leu-Asn-Pro-Phe-Ile- Asn-Arg | |
| 15 | Vimentin | Ser-Pro-Gly-Gly-Val-Tyr-Ala- Thr-Arg-Ser-Ser-Ala-Val-Arg- Leu-Arg | |
| 16 | Vimentin | Asp-Val-Arg-Gln-Gln-Tyr-Glu- Ser-Val-Ala | |

**Table 3. The extracellular tissue peptide sequences and their source of the proteins identifier for each peptide is listed. Thirty-six extracellular tissue peptides are equal to or higher than 4 folds (^{∗}) in tissue salivary gland tumor patients compared to healthy tissue from the same patient.**

| Serial number | Source protein identifier | Peptide sequence | Quantitative peptides Fold change* ≥ 4 |
|---|---|---|---|
| 17 | Vimentin | Ser-Pro-Gly-Gly-Val-Tyr-Ala-Thr- Arg-Ser-Ser-Ala | |
| 18 | Latent-transforming growth factor beta-binding protein 2 | Ser-Thr-Pro-Leu-Gly-Gln-Gln-Gln- Pro-Ala-Pro-Arg | |
| 19 | Vimentin | Val-Glu-Thr-Arg-Asp-Gly-Gln-Val- Ile-Asn-Glu-Thr | |
| 20 | Vimentin | Pro-Gly-Val-Arg-Leu-Leu-Gln-Asp | |
| 21 | Vimentin | Asp-Phe-Ser-Leu-Ala-Asp-Ala | |
| 22 | Vimentin | Ser-Ser-Val-Pro-Gly-Val-Arg-Leu- Leu-Gln | |
| 23 | Vimentin | Thr-Arg-Asp-Gly-Gln-Val-Ile-Asn- Glu | |
| 24 | Vimentin | Gly-Gly-Pro-Gly-Thr-Ala-Ser-Arg- Pro-Ser-Ser-Ser-Arg-Ser-Tyr | |
| 25 | Vimentin | Ser-Ser-Leu-Asn-Leu-Arg-Glu-Thr- Asn | |
| 26 | Interferon-induced transmembrane protein 1 | Met-His-Lys-Glu-Glu-His-Glu-Val- Ala-Val-Leu-Gly | |
| 27 | Vimentin | Val-Glu-Thr-Arg-Asp-Gly-Gln-Val- Ile-Asn-Glu-Thr-Ser-Gln-His-His- Asp-Asp-Leu-Glu | |
| 28 | Hemoglobin subunit alpha | Gly-Lys-Val-Gly-Ala-His-Ala-Gly- Glu-Tyr-Gly-Ala-Glu-Ala-Leu-Glu- Arg-Met | |
| 29 | Vimentin | Ser-Thr-Arg-Ser-Val-Ser-Ser-Ser-Ser- Tyr-Arg-Arg | |
| 30 | Vimentin | Ser-Ala-Leu-Arg-Pro-Ser-Thr-Ser- Arg-Ser-Leu | |
| 31 | Vimentin | Ser-Leu-Asn-Leu-Arg-Glu-Thr-Asn | |
| 32 | Clusterin | Phe-Thr-Arg-Glu-Pro-Gln-Asp-Thr | |
| 33 | Vimentin | Val-Ser-Lys-Pro-Asp-Leu-Thr | |
| 34 | Prolow-density lipoprotein receptor-related protein 1 | Arg-Gly-Pro-Glu-Asp-Glu-Ile-Gly- Asp-Pro-Leu-Ala | |
| 35 | Vimentin | Ser-Ser-Val-Pro-Gly-Val-Arg-Leu- Leu-Gln-Asp-Ser | |
| 36 | Hemoglobin subunit delta | Val-Val-Tyr-Pro-Trp-Thr-Gln-Arg- Phe-Phe-Glu-Ser | |
| 37 | Hemoglobin subunit alpha | Phe-Leu-Ser-Phe-Pro-Thr-Thr-Lys- Thr-Tyr-Phe | |
| 38 | Tubulin alpha-1B chain | Asp-Leu-Glu-Pro-Thr-Val-Ile-Asp- Glu-Val-Arg-Thr-Gly | |
| 39 | Hemoglobin subunit delta | Tyr-Gln-Lys-Val-Val-Ala-Gly-Val- Ala-Asn-Ala-Leu-Ala-His-Lys-Tyr | |
| 40 | Vimentin | Ser-Ser-Val-Pro-Gly-Val-Arg-Leu- Leu-Gln-Asp | |
| 41 | Microtubule-associated protein 4 | Asp-Asp-Val-Val-Gly-Glu-Thr-Val- Gly-Lys-Thr-Asp | |
| 42 | Vimentin | Asp-Ser-Leu-Pro-Leu-Val-Asp-Thr- | |
| | | His-Ser-Lys-Arg | |
| 43 | Microtubule-associated protein RP/EB family member 2 | Ser-Pro-Ala-Ala-Lys-Pro-Gly-Ser- Thr-Pro-Ser-Arg-Pro-Ser-Ser-Ala | |
| 44 | Annexin A1 | Glu-Tyr-Val-Gln-Thr-Val-Lys | |
| 45 | Vimentin | Gln-Val-Ile-Asn-Glu-Thr-Ser-Gln- His-His-Asp-Asp-Leu-Glu | |
| 46 | Annexin A1 | Phe-Ile-Glu-Asn-Glu-Glu-Gln-Glu | |
| 47 | Biglycan | Ala-Ile-Gln-Phe-Gly-Asn-Tyr | |
| 48 | Calponin-3 | Thr-His-Phe-Asn-Lys-Gly-Pro-Ser- Tyr-Gly | |
| 49 | Fibrinogen alpha chain | Ser-His-Ser-Leu-Thr-Thr-Asn-Ile- Met-Glu | |
| 50 | Src substrate cortactin | Val-Pro-Val-Glu-Ala-Val-Thr-Ser- Lys | |
| 51 | Y-box-binding protein 1 | Ala-Ala-Asp-Pro-Pro-Ala-Glu-Asn- Ser-Ser-Ala-Pro-Glu-Ala-Glu-Gln- Gly-Gly-Ala-Glu | |
| 52 | Fibrinogen alpha chain | Ser-Glu-Ser-Gly-Ser-Phe-Arg-Pro- Asp-Ser-Pro-Gly-Ser-Gly-Asn-Ala- Arg-Pro-Asn-Asn-Pro-Asp-Trp-Gly- Thr-Phe | |

To further understand biological significance inferred from tissue extracellular peptidome quantitative data in salivary gland tumor patients, employed Search Tool for the Retrieval of Interacting Genes/Proteins (STRING) was carried out. Initially, generated interactome maps in Search Tool for the Retrieval of Interacting Genes/Proteins (STRING) to determine characteristic nodes enriched in tissue salivary gland tumor patients as compared with healthy tissue from the same patient. STRING aims to discriminate specifically between tissue tumor and healthy samples. String analyses of significantly dysregulated protein identifiers in extracellular matrix proteins or collagen-containing extracellular matrix. Analysis shows interesting STRING nodes of identified sources of peptides with statistically different abundances in salivary gland tumor tissue and healthy tissue samples. The STRING analysis of tissue extracellular peptidome composed of a majority of extracellular sources of detected peptides with high functional connectivity between obtained hits. Thus, interaction networks provide another potent way of determining key enriched protein nodes from which a predictive role of the tissue extracellular peptide could be determined.

The tissue extracellular peptidomics approach allows us to screen the peptide level expression in different tissue conditions. The screening facilitates understanding the exact biological mechanism and the discovery of tissue extracellular peptide biomarkers for various disease conditions. Therefore, the final analysis of tissue extracellular peptidomics shows that differential abundance profile of multiple peptides between the salivary gland tumor patients compared with healthy tissue samples. Invention quantitative data shows even single proteins are the source of various tissue extracellular peptides that may have differential abundance profiles. The quantitative analysis of the intracellular peptides sequenced with tandem mass spectrometry can be performed when signal intensity of the studied peptide is at t-test and permutation-based false discovery rate (FDR) correction at 1% FDR (false discovery rate) between salivary gland tumor with healthy tissue samples. Inventors are able to quantify these fascinating examples, namely Vimentin and Latent-transforming growth factor beta-binding protein 2 (LTBP2) proteins are sources of subsets of differentially abundant peptides detected in salivary gland tumor tissue compared with healthy tissue samples experiment. Analysis of tissue extracellular peptide source distribution and fold change for sources of differentiating peptides, especially amino acid location in the sequence and fold change of detected peptides in the context of their position in full-length protein. Moreover, the difference between sample groups for each peptide and profiles of peptide regulation between comparisons of salivary gland tumor tissue and healthy tissue samples.

Inventors considered the quantitate analysis and the following two peptides as an example to show that the tissue extracellular peptidomics has potential applications towards the tissue derived biomarker discovery and generation of anti-cancer cellular therapies and peptide vaccines. Analysis data revealed two peptides, one derived from Vimentin - an intermediate filament protein typical for mesenchymal cells, and second derived from Latent-transforming growth factor beta-binding protein 2 (LTBP2) - an extracellular matrix protein and growth factor binding protein. Vimentin peptide comes from the N-terminal part of the protein (17 to 34 AA) and is upregulated in salivary gland tumor tissue samples, however after inspecting the data, inventors have found that almost all Vimentin-derived peptides show a similar trend. These peptides are distributed non-randomly on the protein sequence, concentrating in the N-terminal (1-140 AA), C-terminal (410-466 AA), and region in the middle of the sequence (260-320 AA). These peptides may simply represent the pattern of protein degradation, signaling modulation. In the case of LTBP2, the peptide in question was heavily upregulated in salivary gland tumor tissue samples. Inventors were also able to observe that other peptides coming from this protein show identical trend. All of them were derived from a relatively well-defined region located near N-terminus, roughly from 70 to 140 AA. Therefore, the vimentin and LTBP2 derived overexpressed in salivary gland tumor tissue samples indicating to play a role in salivary gland tumor progression and are the potential biomarkers for early diagnosis and peptide patterns dedicated for generation of anti-cancer vaccines against salivary gland tumor, identification and isolation of salivary gland tumor specific T cells in humans. In addition, the tissue extracellular peptidomics approach clearly discriminates the peptides which are differentially regulated and non-regulated peptides even though the source of the protein is the same. It suggests that there are differential isoform degradation, partial degradation or other more complex process concerning these proteins in this cellular model. Therefore, these correlations between different peptides with potentially identical sources can be helpful for the biological interpretation of the results for salivary gland tumor samples. Hence the novel approach for tissue extracellular peptidome sample preparation is comprehensive, qualitative, and quantitative and provides insight into salivary gland tumor biomarker discovery. Quantitative 52 of peptide patterns dedicated for generation of anti-cancer vaccines against salivary gland tumor, identification and isolation of salivary gland tumor specific T cells in humans and diagnostic marker peptides are increased 4 folds or more in the extracellular tissue of the patient with salivary gland tumor as compared with the healthy tissue of the same patient, As grade of the fold increase of the quantitative peptides correlates positively with their predictive significance for peptide patterns dedicated for generation of anti-cancer vaccines against salivary gland tumor, identification and isolation of salivary gland tumor specific T cells in humans and salivary gland tumor diagnostics, inventors also define a tissue extracellular peptide patterns 1, 2, and 3. The extracellular tissue tumor peptide pattern 1 is the list of 2 extracellular tissue peptides that are equal to or higher than 100 folds in tissue salivary gland tumor patients compared to healthy tissue from the same patient. The extracellular tissue tumor peptide pattern 2 is the list of 14 extracellular tissue peptides that are equal to or higher than 20 folds in tissue salivary gland tumor patients compared to healthy tissue from the same patient. The extracellular tissue tumor peptide pattern 3 is the list of 36 extracellular tissue peptides that are equal to or higher than 4 folds in tissue salivary gland tumor patients compared to healthy tissue from the same patient Therefore, it will revolutionize the peptide patterns dedicated for generation of anti-cancer vaccines against salivary gland tumor, identification and isolation of salivary gland tumor specific T cells in humans and early diagnostic field and show a roadmap to the Point of Care Diagnostic (POCD) developments. Ultimately, it will improve the quality of life and be applied to the globe. Therefore, the invention will bring breakthroughs in Point of Care Diagnostic (POCD) platforms and revolutionize the medical field. It is ready to be employed for all kinds (human/non-human) of control/disease tissue samples in the future.

### References

[1] R. A. de Lima-Souza et al., "Protein markers of primary salivary gland tumors: A systematic review of proteomic profiling studies," Arch. Oral Biol., vol. 136, p. 105373, Apr. 2022, doi: 10.1016/j.archoralbio.2022.105373.
[2] M. T. Meyer, C. Watermann, T. Dreyer, S. Ergün, and S. Karnati, "2021 Update on Diagnostic Markers and Translocation in Salivary Gland Tumors," Int. J. Mol. Sci., vol. 22, no. 13, p. 6771, Jun. 2021, doi: 10.3390/ijms22136771.
[3] R. Richter et al., "Composition of the peptide fraction in human blood plasma: database of circulating human peptides," J. Chromatogr. B. Biomed. Sci. App., vol. 726, no. 1, pp. 25-35, Apr. 1999, doi: 10.1016/S0378-4347(99)00012-2.
[4] S. Dasgupta et al., "Proteasome Inhibitors Alter Levels of Intracellular Peptides in HEK293T and SH-SY5Y Cells," PLOS ONE, vol. 9, no. 7, p. e103604, Jul. 2014, doi: 10.1371/journal.pone.0103604.
[5] J. Hu et al., "Peptidomic analysis on synovial tissue reveals galectin-1 derived peptide as a potential bioactive molecule against rheumatoid arthritis," Cytokine, vol. 131, p. 155020, Jul. 2020, doi: 10.1016/j.cyto.2020.155020.
[6] F. Zhang et al., "Peptidome Analysis of Pancreatic Tissue Derived from T1DM Mice: Insights into the Pathogenesis and Clinical Treatments of T1DM," BioMed Res. Int., vol. 2021, p. 9987042, May 2021, doi: 10.1155/2021/9987042.
[7] T. Chen, Z. Zhang, Q. Lu, and J. Ma, "Screening and functional analysis of the differential peptides from the placenta of patients with healthy pregnancy and preeclampsia using placental peptidome," Front. Genet., vol. 13, p. 1014836, 2022, doi: 10.3389/fgene.2022.1014836.

## Claims

1. Tissue extracellular peptides dedicated for generation of anti-cancer vaccines against salivary gland tumor, identification and isolation of salivary gland tumor specific T cells in humans that set a list of the following 52 quantitative peptides Ser-Thr-Pro-Leu-Gly-Gln-Gln-Gln-Pro-Ala-Pro-Arg-Thr-Arg, Ala-Leu-Arg-Pro-Ser-Thr-Ser-Arg-Ser-Leu-Tyr, Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala, Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of the each peptides in extracellular tumor tissue of patient with salivary gland tumor are increased 4 folds or more as compared with healthy tissue from the same patient.

2. The tissue extracellular peptides according to claim 1, wherein tissue extracellular tumor peptide levels of the following 2 quantitative peptides Ser-Thr-Pro-Leu-Gly-Gln-Gln-Gln-Pro-Ala-Pro-Arg-Thr-Arg, and Ala-Leu-Arg-Pro-Ser-Thr-Ser-Arg-Ser-Leu-Tyr wherein the tissue extracellular tumor peptide level of mentioned 2 peptides in extracellular tumor tissue of patient with salivary gland tumor are increased 100 folds or more as compared with healthy tissue from the same patient.

3. The tissue extracellular peptides according to claim 1, wherein tissue extracellular tumor peptide levels of the following 14 quantitative peptides Glu-Thr-Arg-Asp-Gly-Gln-Val-Ile-Asn-Glu-Thr-Ser-Gln, Ser-Ser-Leu-Asn-Leu-Arg-Glu-Thr-Asn-Leu-Asp, Thr-Pro-Leu-Gly-Gln-Gln-Gln-Pro-Ala-Pro-Arg, Ala-Arg-Met-Leu-Ala-Gln-Glu, Ser-Thr-Ser-Arg-Ser-Leu-Tyr, Leu-Val-Asp-Thr-His-Ser-Lys-Arg-Thr-Leu, Phe-Gly-Gly-Pro-Gly-Thr-Ala-Ser-Arg-Pro-Ser-Ser-Ser-Arg-Ser-Tyr-Val-Thr-Thr, Thr-Arg-Asp-Gly-Gln-Val-Ile-Asn-Glu-Thr, Gly-Gly-Pro-Gly-Thr-Ala-Ser-Arg-Pro-Ser-Ser-Ser-Arg-Ser-Tyr-Val-Thr-Thr, Leu-Ile-Lys-Thr-Val-Glu-Thr-Arg-Asp-Gly-Gln-Val-Ile-Asn-Glu-Thr-Ser-Gln-His-His-Asp-Asp, Asp-Ser-Leu-Pro-Leu-Val-Asp-Thr-His-Ser-Lys-Arg-Thr-Leu, Tyr-Glu-Leu-Asn-Pro-Phe-Ile-Asn-Arg, Ser-Pro-Gly-Gly-Val-Tyr-Ala-Thr-Arg-Ser-Ser-Ala-Val-Arg-Leu-Arg, Asp-Val-Arg-Gln-Gln-Tyr-Glu-Ser-Val-Ala wherein the tissue extracellular tumor peptide level of mentioned 14 peptides in extracellular tumor tissue of patient with salivary gland tumor are increased 20 folds or more as compared with healthy tissue from the same patient.

4. The tissue extracellular peptides according to claim 1, wherein tissue extracellular tumor peptide levels of the following 36 quantitative peptides Ser-Pro-Gly-Gly-Val-Tyr-Ala-Thr-Arg-Ser-Ser-Ala, Ser-Thr-Pro-Leu-Gly-Gln-Gln-Gln-Pro-Ala-Pro-Arg, Val-Glu-Thr-Arg-Asp-Gly-Gln-Val-Ile-Asn-Glu-Thr, Pro-Gly-Val-Arg-Leu-Leu-Gln-Asp, Asp-Phe-Ser-Leu-Ala-Asp-Ala, Ser-Ser-Val-Pro-Gly-Val-Arg-Leu-Leu-Gln, Thr-Arg-Asp-Gly-Gln-Val-Ile-Asn-Glu, Gly-Gly-Pro-Gly-Thr-Ala-Ser-Arg-Pro-Ser-Ser-Ser-Arg-Ser-Tyr, Ser-Ser-Leu-Asn-Leu-Arg-Glu-Thr-Asn, Met-His-Lys-Glu-Glu-His-Glu-Val-Ala-Val-Leu-Gly, Val-Glu-Thr-Arg-Asp-Gly-Gln-Val-Ile-Asn-Glu-Thr-Ser-Gln-His-His-Asp-Asp-Leu-Glu, Gly-Lys-Val-Gly-Ala-His-Ala-Gly-Glu-Tyr-Gly-Ala-Glu-Ala-Leu-Glu-Arg-Met, Ser-Thr-Arg-Ser-Val-Ser-Ser-Ser-Ser-Tyr-Arg-Arg, Ser-Ala-Leu-Arg-Pro-Ser-Thr-Ser-Arg-Ser-Leu, Ser-Leu-Asn-Leu-Arg-Glu-Thr-Asn, Phe-Thr-Arg-Glu-Pro-Gln-Asp-Thr, Val-Ser-Lys-Pro-Asp-Leu-Thr, Arg-Gly-Pro-Glu-Asp-Glu-Ile-Gly-Asp-Pro-Leu-Ala, Ser-Ser-Val-Pro-Gly-Val-Arg-Leu-Leu-Gln-Asp-Ser, Val-Val-Tyr-Pro-Trp-Thr-Gln-Arg-Phe-Phe-Glu-Ser, Phe-Leu-Ser-Phe-Pro-Thr-Thr-Lys-Thr-Tyr-Phe, Asp-Leu-Glu-Pro-Thr-Val-Ile-Asp-Glu-Val-Arg-Thr-Gly, Tyr-Gln-Lys-Val-Val-Ala-Gly-Val-Ala-Asn-Ala-Leu-Ala-His-Lys-Tyr, Ser-Ser-Val-Pro-Gly-Val-Arg-Leu-Leu-Gln-Asp, Asp-Asp-Val-Val-Gly-Glu-Thr-Val-Gly-Lys-Thr-Asp, Asp-Ser-Leu-Pro-Leu-Val-Asp-Thr-His-Ser-Lys-Arg, Ser-Pro-Ala-Ala-Lys-Pro-Gly-Ser-Thr-Pro-Ser-Arg-Pro-Ser-Ser-Ala, Glu-Tyr-Val-Gln-Thr-Val-Lys, Gln-Val-Ile-Asn-Glu-Thr-Ser-Gln-His-His-Asp-Asp-Leu-Glu, Phe-Ile-Glu-Asn-Glu-Glu-Gln-Glu, Ala-Ile-Gln-Phe-Gly-Asn-Tyr, Thr-His-Phe-Asn-Lys-Gly-Pro-Ser-Tyr-Gly, Ser-His-Ser-Leu-Thr-Thr-Asn-Ile-Met-Glu, Val-Pro-Val-Glu-Ala-Val-Thr-Ser-Lys, Ala-Ala-Asp-Pro-Pro-Ala-Glu-Asn-Ser-Ser-Ala-Pro-Glu-Ala-Glu-Gln-Gly-Gly-Ala-Glu, Ser-Glu-Ser-Gly-Ser-Phe-Arg-Pro-Asp-Ser-Pro-Gly-Ser-Gly-Asn-Ala-Arg-Pro-Asn-Asn-Pro-Asp-Trp-Gly-Thr-Phe wherein the tissue extracellular tumor peptide level of mentioned 36 peptides in extracellular tumor tissue of patient with salivary gland tumor are increased 4 folds or more as compared with healthy tissue from the same patient.

5. The salivary gland extracellular tumor peptide pattern according to claim 1-4, wherein peptides are detected and measured in salivary gland extracellular tumor samples.

6. The salivary gland extracellular tumor peptide pattern according to claims 1-5, wherein the salivary gland extracellular tumor peptide pattern is defined with tandem mass spectrometry, however the method of peptide identification and quantification is not the limitation of the invention and can be different than tandem mass spectrometry.
